(19) Europäisches Patentamt
European Patent Office
Office européen des brevets

(11) Numéro de publication: **0 002 408**
**A1**

(12) # DEMANDE DE BREVET EUROPEEN

(21) Numéro de dépôt: **78400193.5**

(22) Date de dépôt: **20.11.78**

(51) Int. Cl.[2]: **C 07 C 49/84**
C 07 C 43/20, C 07 C 43/28
C 07 C 45/00, C 07 C 41/04
C 07 C 41/00, A 61 K 31/085
A 61 K 31/12
//C07D317/22, C07C69/67,
C07C67/30

(30) Priorité: **26.11.77 GB 49323/77**

(43) Date de publication de la demande:
**13.06.79 Bulletin 79/12**

(84) Etats contractants désignés:
**BE CH DE FR GB LU NL SE**

(71) Demandeur: **Société Anonyme dite : SOCIETE DE RECHERCHES INDUSTRIELLES S.O.R.I.**
**50 Avenue des Champs Elysées**
**F-75008 Paris(FR)**

(72) Inventeur: **Majoie, Bernard**
**71, rue Montchapet**
**F-21000 Dijon(FR)**

(74) Mandataire: **Clisci, Serge et al,**
**CABINET BEAU DE LOMENIE 55 rue d'Amsterdam**
**F-75008 Paris(FR)**

(54) **Nouveaux composés phénoxy-alcanols substitués, leur procédé de préparation et leur application en thérapeutique.**

(57) La présente invention concerne en tant que produits industriels nouveaux les phénoxy-alcanols substitués de formule

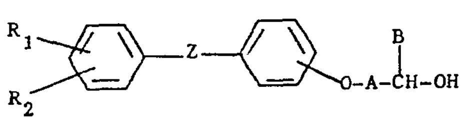

dans laquelle:
- $R_1$ et $R_2$, identiques ou différents, représentent chacun H, F, Cl, Br, $CF_3$, un groupe alkyle inférieur en $C_1-C_3$, ou un groupe alkoxy inférieur en $C_1-C_3$;
- Z représente un groupe carbonyle CO, carbinol CHOH ou méhylène $CH_2$;
- A représente un groupe $CH_2$, $CH(CH_3)$, $C(CH_3)_2$, $CH_2CH_2$, $CH_2CH(CH_3)$, $CH(CH_3)CH_2$, ou $CH_2CH(OH)$;
- B représente H ou $CH_3$; et
- le reste O−A−CHB−OH est toujours en position "méta" ou "para" par rapport au groupe Z, alors qu'en revanche les groupes $R_1$ et $R_2$ peuvent être dans une position quelconque par rapport au même groupe Z.

Elle concerne également le procédé de préparation de ces produits et leur application en thérapeutique.

EP 0 002 408 A1

La présente invention concerne en tant que produits industriels nouveaux des composés phénoxy-alcanols substitués. Elle concerne également leur procédé de préparation et leur application en thérapeutique, notamment en tant qu'agents actifs sur le système nerveux central.

On sait que, dans les brevets belges 742 484, 790 026, 838 435, 845 308 et 853 574, on a proposé, en tant que substances hypocholestérolémiantes et hypolipidémiantes utiles notamment dans le traitement des maladies cardiovasculaires, des acides p-benzoyl-, m-benzoyl-, p-(α-hydroxybenzyl)-, et m-(α-hydroxybenzyl)-phénoxyalkylcarboxyliques, et leurs esters et amides. On vient de trouver que les phénoxy-alcanols substitués de formule I, qui sont structurellement différents des produits connus rappelés ci-dessus, sont des substances utiles en thérapeutique.

Les nouveaux composés phénoxy-alcanols substitués selon l'invention sont caractérisés en ce qu'ils répondent à la formule générale I donnée ci-après, dans laquelle :

- $R_1$ et $R_2$, identiques ou différents, représentent chacun H, F, Cl, Br, $CF_3$, un groupe alkyle inférieur en $C_1$-$C_3$, ou un groupe alkoxy inférieur en $C_1$-$C_3$;
- Z représente un groupe carbonyle CO, carbinol CHOH ou méthylène $CH_2$;
- A représente un groupe $CH_2$, $CH(CH_3)$, $C(CH_3)_2$, $CH_2CH_2$, $CH_2CH(CH_3)$, $CH(CH_3)CH_2$ ou $CH_2CH(OH)$
- B représente H ou $CH_3$; et
- le reste O-A-CHB-OH est toujours en position "méta" ou "para" par rapport au groupe Z, alors qu'en revanche les groupes $R_1$ et $R_2$ peuvent être dans une position quelconque par rapport au même groupe Z.

Parmi les composés préférés inclus dans les définitions données ci-dessus, on peut notamment citer les composés de formules Ia et Ib données ci-après, dans lesquelles $R_1$, $R_2$ et Z sont définis comme ci dessus; Y est H ou $CH_3$; n a pour valeur 0 ou 1; $Y_1$ est H ou OH et peut représenter $CH_3$ lorsque n est 1; et $Y_2$ est H ou OH, avec la condition supplémentaire qu'un au moins des $Y_1$ et $Y_2$ représente le groupe OH.

Les composés de formule I peuvent être préparés selon une méthode connue en soi, par application de mécanismes réactionnels classiques. Selon l'invention, on préconise deux méthodes industriellement intéressantes, selon que A est $CH(CH_3)$ ou $C(CH_3)_2$ (méthode I) ou selon que A est différent de $CH(CH_3)$ et $C(CH_3)_2$ (méthode II). La méthode I s'applique avantageusement à la synthèse des composés Ia et la méthode II à la synthèse des composés Ib.

La méthode I pour la préparation d'un composé Ia consiste à réduire un ester de formule II donnée ci après, dans laquelle R' représente un groupe alkyle inférieur en $C_1$-$C_3$ et $R_1$, $R_2$, Z et Y sont définis comme indiqué ci-dessus, au moyen d'un agent réducteur, dans un solvant organique ayant une température d'ébullition inférieure à 100°C sous 1 atmosphère, la température de réaction étant avantageusement comprise entre 0 et 60°C. Les solvants préférés sont notamment l'éther et le tétrahydrofuranne.

Parmi les agents réducteurs utilisables à cet effet, on peut notamment mentionner $LiAlH_4$, $KBH_4$, les hydrures d'alkyllithium et le réactif de Bouvault et Blanc (sodium et alcool), l'agent réducteur préféré étant $LiAlH_4$.

Les commentaires relatifs à la méthode I sont les suivants.

1°) - Quand Z est $CH_2$ et CHOH, la méthode I est directement utilisable. En revanche, quand Z est CO, il faut bien sûr commencer par protéger la fonction carbonyle [notamment sous la forme d'un groupe 2-(1,3-dioxolannyle)] avant de faire réagir l'agent réducteur. La réduction de la fonction COOR' en $CH_2OH$ étant réalisée, on régénère alors la fonction carbonyle Z=CO [notamment par traitement en milieu légèrement acide lorsque le groupe protecteur de la fonction acide est le groupe 2-(1,3-dioxolannyle)].

2°) - Bien entendu, il est possible d'envisager la préparation d'un composé Ia, dans lequel Z est CHOH, à partir d'un composé II dans lequel Z est CO, par réduction simultanée de la fonction carbonyle CO et de la fonction ester COOR'; cependant, on préfère plutôt :

a) - soit appliquer la méthode I à partir d'un composé de formule II où Z est CHOH,

b) - soit réduire un composé II où Z est CO au borohydrure de potassium (agent de réduction doux), afin de transformer Z en CHOH, puis réduire la fonction ester,

afin de ne réduire qu'une seule fonction à la fois.

La méthode II pour la synthèse d'un composé de formule Ib consiste à faire réagir un phénol substitué de formule III donnée ci-après où $R_1$, $R_2$ et Z sont définis comme indiqué ci-dessus, avec un halogéno-alcanol de formule IV donnée ci-après, où n, $Y_1$ et $Y_2$ sont définis comme indiqué ci-dessus et X représente un atome d'halogène, notamment le chlore ou le brome (le chlore étant l'halogène préféré). Cette réaction est effectuée au reflux, en présence de $K_2CO_3$ au sein de l'isopropanol ou d'un autre alcool ayant une température d'ébullition comprise entre 60°C et 130°C, sous 1 atmosphère, le reflux étant maintenu pendant 6 à 45 h. L'alcool préféré est l'isopropanol.

Dans le tableau I ci-après, on a consigné, de façon non limitative, un certain nombre de composés de formule I selon l'invention. Dans ce tableau, la colonne "type" concerne la position (para ou méta) du reste O-A-CHB-OH par rapport au groupe Z et la position des substituants $R_1$ et $R_2$ est repérée par rapport au même groupe Z.

Les composés de formule I sont utiles en tant que médicaments. Leur propriété commune réside dans le fait qu'ils agissent sur le SNC, notamment en tant qu'agents anxiolytiques. A côté de cette propriété commune, certains composés, en particulier les composés Ia, présentent une action hypocholestérolémiante et hypolipidémiante, et certains autres composés, en particulier les composés Ib, présentent une action uricosurique.

TABLEAU I

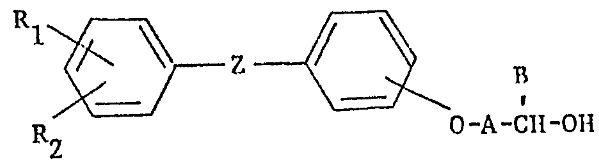

| Exemple | n° de code | Type | A | B | Z | $R_1$ | $R_2$ | Point de fusion F. ou indice de réfraction $n_D^{20}$ |
|---|---|---|---|---|---|---|---|---|
| 1 | 853 | para | $C(CH_3)_2$ | H | CO | $4-CF_3$ | H | F = 64°C |
| 2 | 854 | para | $C(CH_3)_2$ | H | CHOH | $4-CF_3$ | H | F = 112°C |
| 3 | 795 | para | $C(CH_3)_2$ | H | CO | 4-Cl | H | F = 74°C |
| 4 | 542 | para | $C(CH_3)_2$ | H | CHOH | 4-Cl | H | F = 95°C |
| 5 | 845 | para | $CH(CH_3)$ | H | CO | 4-Cl | H | F = 66°C |
| 6 | 859 A | para | $C(CH_3)_2$ | H | CO | H | H | F = 62°C |
| 7 | 859 | para | $C(CH_3)_2$ | H | CHOH | H | H | F = 110°C |
| 8 | 860 A | para | $C(CH_3)_2$ | H | CO | $4-CH_3O$ | H | F = 69°C |
| 9 | 860 | para | $C(CH_3)_2$ | H | CHOH | $4-CH_3O$ | H | F = 89°C |
| 10 | 861 | para | $C(CH_3)_2$ | H | CO | 4-Br | H | F = 71°C |
| [illegible] | [illegible] | [illegible] | [illegible] | H | CHOH | 4-Br | H | F = 91°C |
| 12 | 826 | méta | $C(CH_3)_2$ | H | CO | $4-CF_3$ | H | n = 1,522 |
| 13 | 881 | para | $C(CH_3)_2$ | H | CO | 4-F | H | F = 60°C |
| 14 | 890 | para | $CH(CH_3)$ | H | CHOH | 4-Cl | H | n = 1,585 |

TABLEAU I (suite 1)

| Exemple | n° de code | Type | A | B | Z | $R_1$ | $R_2$ | Point de fusion F. ou indice de réfraction $n_{20}^{D}$ |
|---|---|---|---|---|---|---|---|---|
| 15 | 891 | méta | $CH(CH_3)$ | H | CHOH | 4-Cl | 2-Cl | n = 1,585 |
| 16 | 892 | méta | $C(CH_3)_2$ | H | CHOH | 4-Cl | 2-Cl | F = 87°C |
| 17 | 891A | méta | $CH(CH_3)$ | H | CO | 4-Cl | 2-Cl | n = 1,582 |
| 18 | 906 | méta | $C(CH_3)_2$ | H | CO | 4-Cl | 2-Cl | n = 1,584 |
| 19 | 935A | méta | $C(CH_3)_2$ | H | $CH_2$ | $4-CF_3$ | H | F = 65°C |
| 20 | 935 | para | $C(CH_3)_2$ | H | $CH_2$ | 4-Cl | H | F = 46°C |
| 21 | 838 | para | $CH_2$ | H | CO | 4-Cl | H | F = 132°C |
| 22 | 880 | para | $CH_2$ | H | CHOH | 4-Cl | H | F = 83°C |
| 23 | 771 | para | $CH_2CH_2$ | H | CO | 4-Cl | H | F = 128°C |
| 24 | 867 | para | $CH_2CH(OH)$ | H | CO | 4-Cl | H | F = 154°C |
| 25 | 870 | méta | $CH_2$ | H | CO | $4-CH_3$ | H | F = 80°C |
| 26 | 869 | méta | $CH_2CH(OH)$ | H | CO | $4-CH_3$ | H | F = 100°C |
| 27 | 895 | para | $CH_2$ | $CH_3$ | CO | 4-Cl | H | F = 122°C |
| 28 | 924A | para | $CH_2CH_2$ | H | CHOH | 4-Cl | H | F = 108°C |
| 29 | 870A | méta | $CH_2$ | H | CHOH | $4-CH_3$ | H | n = 1,583 |
| 30 | 923A | para | $CH_2CH(OH)$ | H | $CH_2$ | 4-Cl | H | F = 90°C |

TABLEAU I (suite 2)

| Exemple | n° de code | Type | A | B | Z | $R_1$ | $R_2$ | Point de fusion F. indice de réfraction $n_{20}^{D}$ |
|---|---|---|---|---|---|---|---|---|
| 31 | 909 | para | $C(CH_3)_2$ | H | CHOH | 4-$CH_3$ | H | F = 98°C |
| 32 | 912 | para | $C(CH_3)_2$ | H | CO | 4-$CH_3$ | H | F = 51°C |
| 33 | 923 | méta | $CH_2CHOH$ | H | CO | 4-Cl | 2-Cl | F = 50°C |
| 34 | 924 | para | $CH_2CH_2$ | H | CO | 4-Cl | 2-Cl | F = 81°C |
| 35 | 925 | méta | $CH_2CH_2$ | H | CO | 4-Cl | 2-Cl | F = 47,5°C |
| 36 | 928 | para | $CH_2$ | $CH_3$ | CO | 4-Cl | 2-Cl | n = 1,608 |
| 37 | 934 | para | $C(CH_3)_2$ | H | $CH_2$ | 4-$CH_3$ | H | F = 47°C |
| 38 | 936 | para | $CH(CH_3)$ | H | $CH_2$ | 4-Cl | H | n = 1,570 |
| 39 | 943 | para | $C(CH_3)_2$ | H | $CH_2$ | H | H | n = 1,555 |
| 40 | 944 | para | $CH_2$ | H | CO | 4-Cl | 2-Cl | F = 83°C |
| 41 | 991 | méta | $CH(CH_3)$ | H | CO | H | H | n = 1,583 |
| 42 | 1228 | méta | $C(CH_3)_2$ | H | $CH_2$ | H | H | n = 1,560 |

Les composés de formule I peuvent être administrés notamment par voie injectable ou par voie orale.

Selon l'invention, on préconise une composition thérapeutique caractérisée en ce qu'elle renferme, en association avec un excipient physiologiquement acceptable, au moins un composé de formule I comme ingrédient actif.

D'autres avantages et caractéristiques de l'invention seront mieux compris à la lecture qui va suivre d'exemples de préparations nullement limitatifs mais donnés à titre d'illustration.

PREPARATION I

Obtention du 2-[4-(4-trifluorométhylbenzoyl)-phénoxy]-2-méthyl-1-propanol.

n° de code : 853 (exemple 1, tableau I)

Etape a

Dans un tricol de 500 ml, on introduit, sous azote, 10 g (0,025 mole) de 2-[4-(4-trifluorométhylbenzoyl)-phénoxy]-2-méthylpropionate d'isopropyle, 6% en poids par rapport au poids de l'ester précédent (soit 0,60 g) d'acide paratoluènesulfonique, 25 ml de $HOCH_2CH_2OH$ et 150 ml de toluène. Ce mélange réactionnel est porté au reflux sous agitation et l'on suit l'élimination de l'eau formée, le reflux étant maintenu pendant 8 h. On refroidit jusqu'à la température ambiante (15-25°C) et on laisse sous azote, sans agiter, pendant une nuit. On extrait à l'éther, lave la phase éthérée à l'eau, sèche, décolore sur noir de carbone, filtre et évapore sous vide. Le produit attendu cristallise. Par recristallisation dans l'hexane, on obtient 12 g (rendement : 95%) de composé 2-(1,3-dioxolannyle) de formule V donnée ci-après. F. = 68°C.

Etape b

Dans un ballon de 500 ml, on introduit sous azote à 0°C 1 g (0,026 mole) de $LiAlH_4$ et 80 ml d'éther anhydre (il convient de refroidir à 0°C si, lors du mélange, la température s'élève). On ajoute goutte à goutte 8,76 g (0,02 mole) de composé 2-(1,3-dioxolannyle) de formule V en solution dans 20 ml d'éther anhydre. On maintient le milieu réactionnel sous agitation à 0°C pendant 2 h 30 mn. On hydrolyse par de l'éther saturé d'eau puis par 15 ml d'eau glacée. On obtient le composé dioxannyle de formule VI (donnée ci-après) qui cristallise.

BAD ORIGINAL

0002408

Etape c

On dissout le composé VI, obtenu comme indiqué ci-dessus, dans de l'éther. On ajoute ensuite 50 ml d'acide HCl 5N. On agite pendant 12 h à la température ambiante (15-25°C). On extrait à l'éther, lave la phase éthérée à l'eau, sèche, passe sur noir de carbone, filtre, évapore sous vide. Par recristallisation dans l'éther diisopropylique, on obtient 6,50 g (rendement des étapes b et c : 96,2%) de 2-[4-(4-trifluorométhyl-benzoyl)-phénoxy]-2-méthyl-1-propanol. F. = 64°C.

Analyse :

Calculé pour $C_{18}H_{17}F_3O_3$ : C 63,9%; H 5,1%

Trouvé : C 63,9%; H 5,2%.

PREPARATION II

Obtention du 2-[4-(α-hydroxy-4-trifluorométhylbenzyl)-phénoxy]-2-méthyl-1-propanol.

n° de code : 854 (cf. exemple 2 du tableau I et formule VII)

Etape a

Dans un ballon de 500 ml, on introduit, sous azote, 7,10 g (0,018 mole) de 2-[4-(4-trifluorométhylbenzoyl)-phénoxy]-2-méthylpropionate d'isopropyle, 1,46 g (0,027 mole) de $KBH_4$ et 100 ml d'éthanol. On porte au reflux (jusqu'à disparition de la matière première) pendant 1 h 30 mn. On refroidit jusqu'à la température ambiante (15-25°C). On hydrolyse, extrait à l'éther, lave la phase éthérée à l'eau, sèche, passe au noir de carbone, filtre et évapore sous vide. On obtient 6,70 g (rendement 94%) de 2-[4-(α-hydroxy-4-trifluorométhylbenzyl)-phénoxy]-2-méthylpropionate d'isopropyle.

Etape b

Dans un ballon de 500 ml, on fait, sous azote, un mélange de 0,80 g de $LiAlH_4$ et de 50 ml d'éther anhydre et on refroidit à 0°C. On introduit alors goutte à goutte, sous azote, et à 0°C 5,8 g (0,0147 mole) du produit obtenu à l'étape a en solution dans 20 ml d'éther anhydre. On laisse réagir sous agitation à 0°C pendant 3 h. On hydrolyse à 0°C au moyen d'éther saturé en eau puis par de l'eau glacée. On extrait à l'éther, lave la phase éthérée à l'eau, sèche, passe sur noir de carbone, filtre et évapore sous vide. Par recristallisation dans l'éther diisopropylique, on obtient 4,80 g (rendement 96%) de 2-[4-(α-hydroxy-4-trifluorométhyl-benzyl)-phénoxy]-2-méthyl-1-propanol. F. = 112°C.

Analyse :

Calculé pour $C_{18}H_{19}F_3O_3$ : C 63,5%; H 5,6%

Trouvé : C 63,5%; H 5,8%.

PREPARATION III

Obtention du composé de formule VII par voie directe.

On réduit directement le 2-[4-(4-trifluorométhylbenzoyl)-phénoxy]-2-méthylpropionate d'isopropyle (1 mole) au moyen de $LiAlH_4$ (2 moles) dans de l'éther anhydre au reflux pendant 4 h. On obtient le composé de formule VII (F. = 112°C) mais avec un rendement inférieur à 30%. Aussi, il est plutôt recommandé de ne faire qu'une seule réduction à la fois, comme indiqué plus haut, et de mettre en oeuvre la préparation II ou la préparation IV qui suit.

PREPARATION IV

Obtention du composé de formule VII par réduction du dérivé carbonyle (Z=CO) correspondant.

On réduit le 2-[4-(4-trifluorométhylbenzoyl)-phénoxy]-2-méthyl-1-propanol au moyen de $KBH_4$ selon les modalités opératoires de la préparation IIa. On obtient ainsi le composé VII (F. = 112°C) avec un rendement de l'ordre de 94%.

PREPARATION V

Obtention du 2-[4-(4-chlorobenzoyl)-phénoxy]-1-éthanol.

n° de code : 838 (cf. exemple 21 du tableau I)

Dans un ballon de 500 ml, on place 23,2 g (0,1 mole) de 4'-chloro-4-hydroxy-benzophénone, 15 g (0,11 mole) de carbonate de potassium finement divisé et 150 ml d'alcool (isopropanol). On chauffe 1 h à reflux et, tout en maintenant le reflux, on additionne au goutte-à-goutte une solution de 12 g (0,15 mole) de chlorhydrine du glycol diluée dans 50 ml d'isopropanol. Le reflux est maintenu 16 h après la fin de l'addition.

On laisse refroidir, évapore l'isopropanol, reprend le résidu à la soude diluée à 1% (10 g/l de NaOH) en restant en milieu basique, et extrait à l'éther plusieurs fois. Les phases éthérées réunies sont lavées à l'eau jusqu'à neutralité; on les sèche, les décolore au noir animal et les concentre sous vide, on obtient 16 g d'un résidu solide que l'on recristallise dans l'isopropanol. On obtient 14 g de 2-[4-(4-chlorobenzoyl)-phénoxy]-1-éthanol. F. = 132°C.

Analyse :

Calculé pour $C_{15}H_{13}ClO_3$ : C 65,1%; H 4,7%

Trouvé : C 65,4%; H 4,6%.

PREPARATION VI

Obtention du 2-[4-(α-hydroxy-4-chlorobenzyl)-phénoxy]-1-éthanol.

n° de code : 880 (cf. exemple 22 du tableau I)

Dans un ballon de 250 ml, on place 13,5 g (0,05 mole) du produit de l'exemple 21, préparé selon la méthode de la préparation V, dissous dans 100 ml de méthanol, à température ambiante, on ajoute 2,7 g (0,05 mole) de borohydrure de potassium et on laisse la réaction se poursuivre pendant 7 h. Le méthanol est évaporé sous vide, le résidu repris à l'eau et l'éther. La phase éthérée est lavée à l'eau jusqu'à neutralité, séchée, décolorée sur noir de carbone puis concentrée sous vide. Le résidu solide est recristallisé dans l'éthanol . On obtient 13 g de 2-[4-(α-hydroxy-4-chlorobenzyl)-phénoxy]-1-éthanol. F. = 83°C.

Analyse :

Calculé pour $C_{15}H_{15}ClO_3$ : C 64,6%; H 5,4%

Trouvé : C 64,5%; H 5,4%.

PREPARATION VII

Obtention du 3-[4-(4-chlorobenzoyl)-phénoxy]-1,2-propanediol.

n° de code : 867 (cf. exemple 24 du tableau I)

En appliquant le mode opératoire décrit pour la préparation V, mais en remplaçant la chlorhydrine du glycol par la chlorhydrine du glycérol comme réactif halogéné du type IV, on obtient le 3-[4-(4-chlorobenzoyl)-phénoxy]-1,2-propanediol. F. = 154°C.

Analyse :

Calculé pour $C_{16}H_{15}ClO_4$ : C 62,6%; H 4,9%

Trouvé : : C 62,4%; H 5,0%.

0002408

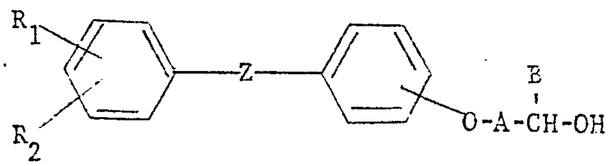
(I)

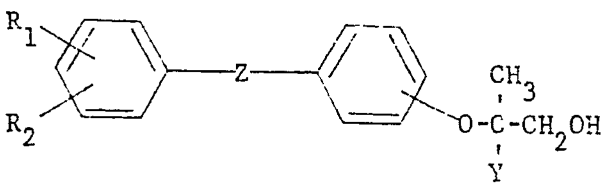
(Ia)

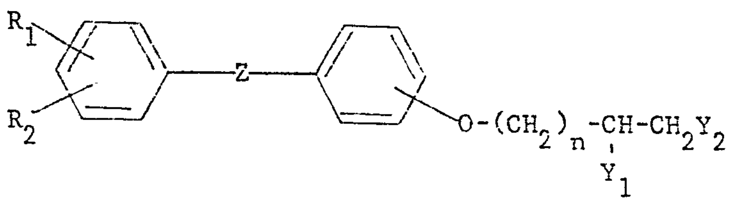
(Ib)

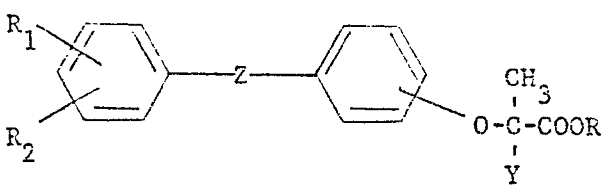
(II)

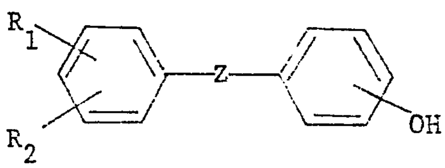
(III)

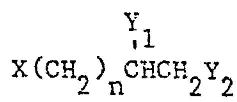
(IV)

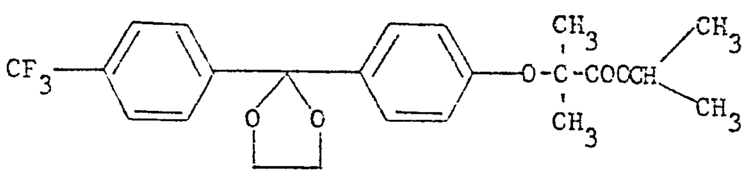
(V)

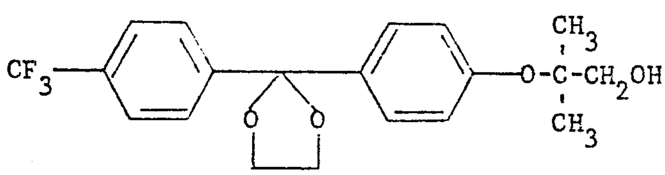
(VI)

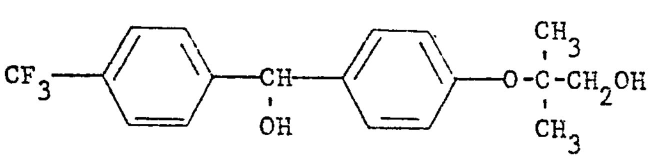
(VII)

## REVENDICATIONS

1 - Nouveau composé phénoxy-alcanol substitué, utile notamment en thérapeutique, caractérisé en ce qu'il répond à la formule générale

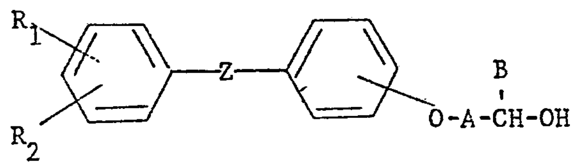

(I)

dans laquelle :

- $R_1$ et $R_2$, identiques ou différents, représentent chacun H, F, Cl, Br, $CF_3$, un groupe alkyle inférieur en $C_1$-$C_3$, ou un groupe alkoxy inférieur en $C_1$-$C_3$;
- Z représente un groupe carbonyle CO, carbinol CHOH ou méthylène $CH_2$;
- A représente un groupe $CH_2$, $CH(CH_3)$, $C(CH_3)_2$, $CH_2CH_2$, $CH_2CH(CH_3)$, $CH(CH_3)CH_2$, ou $CH_2CH(OH)$ ;
- B représente H ou $CH_3$; et
- le reste O-A-CHB-OH est toujours en position "méta" ou "para" par rapport au groupe Z, alors qu'en revanche les groupes $R_1$ et $R_2$ peuvent être dans une position quelconque par rapport au même groupe Z.

2 - Nouveau composé selon la revendication 1, caractérisé en ce qu'il répond à la formule

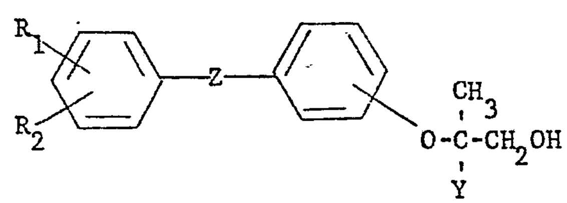

(Ia)

dans laquelle $R_1$, $R_2$ et Z sont définis comme ci-dessus, et Y représente H ou $CH_3$, le groupe $O-CY(CH_3)-CH_2OH$ étant toujours en position "méta" ou "para" par rapport au groupe Z.

3 - Nouveau composé selon la revendication 1, caractérisé en ce qu'il répond à la formule

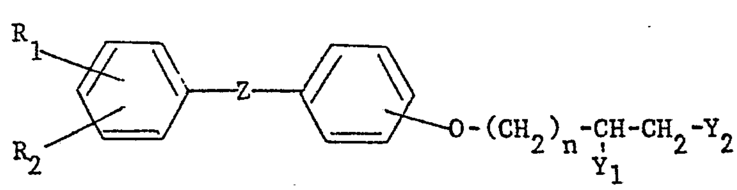

(Ib)

dans laquelle $R_1$, $R_2$ et Z sont définis comme ci-dessus; n a pour valeur 0 ou 1; $Y_1$ est H ou OH et peut représenter $CH_3$ lorsque n est 1; et $Y_2$ est H ou OH, avec la condition supplémentaire qu'au moins un des $Y_1$ et $Y_2$ représente OH, et que le groupe $O-(CH_2)_n-CHY_1-CH_2-Y_2$ soit toujours en position "méta" ou "para" par rapport au groupe Z.

4 - Composé selon la revendication 2, caractérisé en ce qu'il s'agit du 2-[4-(4-trifluorométhylbenzoyl)-phénoxy]-2-méthyl-1-propanol.

5 - Composé selon la revendication 2, caractérisé en ce qu'il s'agit du 2-[4-(α-hydroxy-4-trifluorométhylbenzyl)-phénoxy]-2-méthyl-1-propanol.

6 - Composé selon la revendication 2, caractérisé en ce qu'il s'agit du 2-[3-(benzyl)-phénoxy]-2-méthyl-1-propanol.

7 - Composé selon la revendication 3, caractérisé en ce qu'il s'agit du 2-[4-(4-chlorobenzoyl)-phénoxy]-1-éthanol.

8 - Composé selon la revendication 3, caractérisé en ce qu'il s'agit du 2-[4-(α-hydroxy-4-chlorobenzyl)-phénoxy]-1-éthanol.

9 - Composé selon la revendication 3, caractérisé en ce qu'il s'agit du 3-[4-(4-chlorobenzoyl)-phénoxy]-1,2-propanediol.

10 - Composition thérapeutique, caractérisée en ce qu'elle renferme en association avec un excipient physiologiquement acceptable au moins un composé de formule I selon la revendication 1.

11 - Procédé de préparation d'un composé de formule

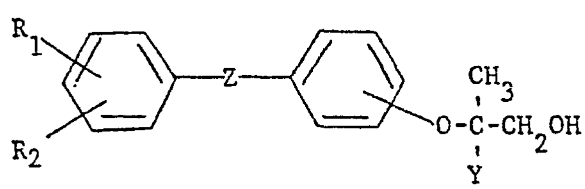

(Ia)

dans laquelle $R_1$, $R_2$ et Z sont définis comme ci-dessus, et Y représente H ou $CH_3$, le groupe $O-CY(CH_3)-CH_2OH$ étant toujours en position "méta" ou "para" par rapport au groupe Z, ledit procédé étant caractérisé en ce que l'on soumet à une réaction de réduction un ester de formule

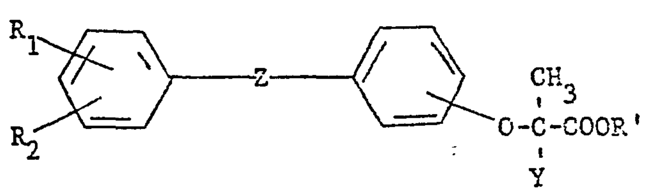

(II)

(où R' est un groupe alkyle en $C_1$-$C_3$), au moyen d'un agent réducteur, dans un solvant organique ayant une température d'ébullition inférieure à 100°C, sous 1 atmosphère, la température de réaction étant comprise entre 0 et 60°C.

12- Procédé de préparation d'un composé de formule

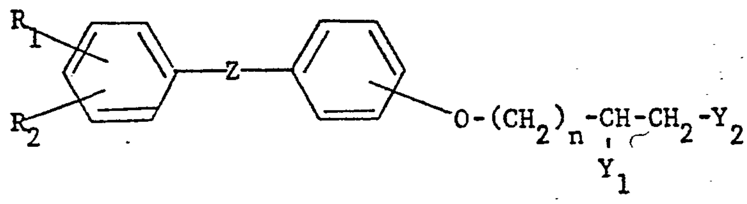

(Ib)

dans laquelle $R_1$, $R_2$ et Z sont définis comme ci dessus; n a pour valeur 0 ou 1; $Y_1$ et H ou OH et peut représenter $CH_3$ lorsque n est 1; et $Y_2$ est H ou OH, avec la condition supplémentaire qu'au moins un des $Y_1$ et $Y_2$ représente OH et que le groupe $O-(CH_2)_n-CHY_1-CH_2-Y_2$ soit toujours en position "méta" ou "para" par rapport au groupe Z; ledit procédé étant caractérisé en ce que l'on fait réagir un phénol substitué de formule

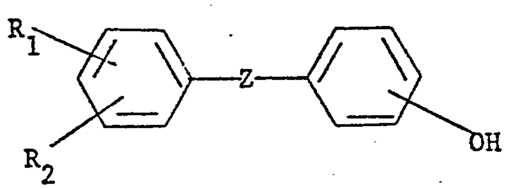

(III)

avec un halogénoalcanol de formule

$$X-(CH_2)_n-\underset{Y_1}{CH}-CH_2-Y_2 \qquad (IV)$$

(où n, $Y_1$ et $Y_2$ sont définis comme ci-dessus, et X représente un atome d'halogène), dans un solvant choisi parmi l'isopropanol et les alcools ayant une température d'ébullition comprise entre 60 et 130°C sous 1 atmosphère, à la température de reflux dudit solvant, en présence de $K_2CO_3$, pendant une durée de 6 à 45 h.

0002408

Office européen des brevets

# RAPPORT DE RECHERCHE EUROPEENNE

Numéro de la demande

EP 78 40 0193

| DOCUMENTS CONSIDERES COMME PERTINENTS | | | CLASSEMENT DE LA DEMANDE (Int. Cl.²) |
|---|---|---|---|
| Catégorie | Citation du document avec indication, en cas de besoin, des parties pertinentes | Revendication concernée | |
| X | DE - A - 2 502 154 (CIBA-GEIGY)<br>* Pages 6,7 *<br>-- | 1 | C 07 C 49/84<br>43/20<br>43/28<br>45/00<br>41/04<br>41/00<br>A 61 K 31/085<br>31/12//<br>C 07 D 317/22<br>C 07 C 69/67<br>67/30 |
| | FR - A - 2 268 774 (CIBA-GEIGY)<br>* Revendication 1; page 3, formule X *<br>---- | 1 | |

DOMAINES TECHNIQUES RECHERCHES (Int. Cl.²)

C 07 C 49/84
43/20
43/28

CATEGORIE DES DOCUMENTS CITES

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

X Le présent rapport de recherche a été établi pour toutes les revendications

| Lieu de la recherche | Date d'achèvement de la recherche | Examinateur |
|---|---|---|
| La Haye | 07-03-1979 | SAGATYS |